# EUROPÄISCHE PATENTSCHRIFT

(11) **EP 0 545 151 B1**
(45) Veröffentlichungstag und Bekanntmachung des Hinweises auf die Patenterteilung: **28.02.1996**
(21) Anmeldenummer: 92119691.1
(22) Anmeldetag: 19.11.1992
(51) Int. Cl.: C07C 209/18

(54) **Verfahren zur Herstellung von N,N-dialkylarylaminen**
Process for the preparation of N,N-dialkylarylamines
Procédé pour la préparation de N,N-dialkylarylamines

(30) Priorität: 29.11.1991 DE 4139362
(43) Veröffentlichungstag der Anmeldung: 09.06.1993
(73) Patentinhaber: BASF Aktiengesellschaft, D-67063 Ludwigshafen (DE)
(72) Erfinder: Reuter, Peter, Dr., W-6800 Mannheim 1 (DE); Weber, Willi Albert, W-6700 Ludwigshafen (DE); Diem, Hans, Dr., W-6800 Mannheim 1 (DE)

(56) Entgegenhaltungen:
- DE-A- 2 348 738
- DE-A- 2 658 728
- DE-B- 1 031 796
- CHEMICAL ABSTRACTS, no. 4, 25. Juli 1991, Columbus, Ohio, US; abstract no. 31575p, Seite 119 ;Spalte 1 ;
- 'HOUBEN-WEYL, METHODEN DER ORGANISCHEN CHEMIE; BAND XI/1' 1957 , STUTTGART

## Beschreibung

Die vorliegende Erfindung betrifft ein verbessertes Verfahren zur Herstellung von N,N-Dialkylarylaminen (I) durch Umsetzung von Arylaminen (II) mit Alkoholen (IIIa) oder Dialkylethern (IIIb) in Gegenwart einer Sauerstoffsäure des Phosphors oder eines Ammoniumsalzes hiervon als Katalysator.

Außerdem betrifft die Erfindung die Regenerierung des Katalysators im Zuge der Herstellung von (I) aus (II) und (IIIa) bzw. (IIIb).

N,N-Dialkylarylamine sind bekanntlich wertvolle Zwischenprodukte für die Herstellung von Pflanzenschutzmitteln und anderen biologischen Wirkstoffen wie Wachstumsregulatoren. Darüber hinaus dienen sie als Ausgangsmaterialien für die Synthese von Pharmazeutika und Farbstoffen, insbesondere den Azofarbstoffen, sowie als Zusatzstoffe für Lacke und Mineralöle.

Aus der DE-B 1 031 796 ist es bekannt, ein Gemisch aus einem aromatischen Amin und einem Alkohol durch heiße konzentrierte Phosphorsäure zu leiten, um auf diese Weise eine N-Alkylierung von Anilin in flüssiger Phase zu bewirken. Der Nachteil dieses Verfahrens besteht darin, daß neben den N,N-Dialkylverbindungen stets noch erhebliche Anteile an N-Monoalkylverbindungen als unerwünschte Nebenprodukte entstehen. Weitere Nachteile sind, daß die Phosphorsäure nach längerer Betriebsdauer an ihrer katalytischen Aktivität einbüßt, die Umsätze zurückgehen und Kernalkylierung als Nebenreaktion stattfindet.

In der DE-A 2 658 728 wird die N-Alkylierung von aromatischen Aminen in Gegenwart von erhitzter Phosphorsäure, der vor oder nach Beginn der Umsetzung ein aliphatisches Amin, ein phosphorsaures Salz oder ein quartäres Ammoniumsalz des Amins zugesetzt wird, beschrieben. Anstelle von Phosphorsäure können auch Metaphosphorsäure und Polyohosphorsäuren verwendet werden. Nachteilig auf die Aktivität wirken sich die in einer unerwünschten Nebenreaktion ablaufende Alkylierung des aliphatischen Amins sowie eine nicht homogene Verteilung der Salze in der Katalysatorphase aus.

Der vorliegenden Erfindung lag daher als Aufgabe zugrunde, den geschilderten Mängeln abzuhelfen und ein selektives, nahezu nebenproduktfreies Verfahren zur Herstellung der N,N-Dialkylarylamine (I) bereitzustellen. Als weitere Aufgabe lag der Erfindung zugrunde, den Katalysator für die Herstellung der N,N-Dialkylarylamine (I) auf einfache und wirtschaftliche Weise zu regenerieren, d.h. ohne verfahrenstechnischen Aufwand seine ursprüngliche Aktivität und Produktivität soweit wie möglich wieder herzustellen.

Demgemäß wurde ein verbessertes Verfahren zur Herstellung von N,N-Dialkylarylaminen (I) durch Umsetzung von Arylaminen (II) mit Alkoholen (IIIa) oder Dialkylethern (IIIb) in Gegenwart einer Sauerstoffsäure des Phosphors oder eines Ammoniumsalzes hiervon als Katalysator gefunden, welches dadurch gekennzeichnet ist, daß man die Sauerstoffsäure des Phosphors vor Beginn der Reaktion mit einem Arylamin (IV) umsetzt.

Weiterhin wurde ein Verfahren zur Herstellung von N,N-Dialkylarylaminen (I) durch Umsetzung von Arylamlnen (II) mit Alkoholen (IIIa) oder Dialkylethern (IIIb) in Gegenwart einer Sauerstoffsäure des Phosphors oder eines Ammoniumsalzes hiervon als Katalysator gefunden, welches dadurch gekennzeichnet ist, daß man die Sauerstoffsaüre des Phosphors vor Beginn der Reaktion mit einem Arylamin (IV) umsetzt und daß die Reaktion von Zeit zu Zeit unterbricht und die Katalysatorphase bei 100 bis 300°C mit Wasserdampf oder Wasser behandelt.

Als Arylamine (IV) eignen sich ganz besonders die N,N-Dialkylarylamine (I), d.h. die Alkylierungsprodukte der Ausgangsarylamine (II). Auch die Ausgangsarylamine (II) kommen für die Katalysatorbereitung in Betracht. Sonstige Arylamine sind zwar ebenfalls geeignet, bedingen aber als synthesefremde Stoffe größeren verfahrenstechnischen Reinigungsaufwand.

Für die Herstellung von N,N-Dimethylanilin aus Anilin und Methanol eignet sich daher vor allem N,N-Dimethylanilin als Amin (IV) sowie daneben auch Anilin.

Allgemein empfehlen sich als Amin (IV) solche der Formel (IVa) 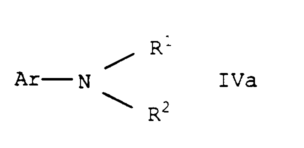
in der
- Ar: einen Arylrest wie Phenyl oder Naphthyl und
- R¹ und R²: Wasserstoff oder C₁-C₁₀-, vorzugsweise C₁-C₆-Alkylgruppen
bedeuten.

Die Reste Ar können einen oder mehrere Substituenten tragen, beispielsweise Alkyl- oder Alkoxygruppen oder Halogene.

Als Sauerstoffsäuren des Phosphors, die mit dem Amin (IV) vorbehandelt werden, eignen sich Orthophosphorsäure und besonders die Anhydrosäuren der Orthophosphorsäure wie vor allem Metaphosphorsäure. In der Regel findet die Kondensation der Sauerstoffsäure des Phosphors und ihre damit verbundene Entwässerung bei Temperaturen zwischen 120 und 270°C statt, wobei eine Temperatur zwischen 160 und 230°C, insbesondere zwischen 200 und 210°C besonders bevorzugt ist.

Die Umsetzung der Sauerstoffsäure des Phosphors erfolgt zweckmäßigerweise mit zuvor außerhalb des Reaktors verdampftem gasförmigem Arylamin (IV) und liefert eine unter den Reaktionsbedingungen schwerflüchtige aktive Katalysatorphase, deren Molverhältnis von Arylamin (IV) zum Phosphorgehalt der Sauerstoffsäure des Phosphors 0,1:1 bis 2:1, vorzugsweise 0,3:1 bis 1,5:1 beträgt. Das Arylamin (IV) kann auch solange durch die Sauerstoffsäure geleitet werden, bis ihre Aufnahmekapazität erschöpft ist, und es im Reaktoraustrag als Gas nachweisbar ist.

Gleiches gilt für die Ammoniumsalze der Sauerstoffsäuren des Phosphors.

Nachdem der Katalysator erfindungsgemäß hergestellt ist, nimmt man die daran anschließende Alkylierung der Arylamine (II) wie üblich vor, indem man das Arylamin (II) und den Alkohol (IIIa) bzw. den Dialkylether (IIIb) gasförmig durch die Katalysatorphase leitet. Zuvor ist es möglich, die beiden Ausgangskomponenten außerhalb des Reaktors auf die gewünschte Reaktionstemperatur zu erhitzen. Das austretende gasförmige Produktgemisch wird in üblicher Weise aufgearbeitet. Nach der Kondensation und Abtrennung der nicht umgesetzten gasförmigen Ausgangsstoffe, die wieder der Reaktion zugeführt werden können, wird das flüssige Produktgemisch durch Phasentrennung in die wäßrige und in die Rohproduktphase zerlegt. Zur weiteren Aufarbeitung kann die Rohproduktphase einer Fraktionierkolonne zugeführt werden.

Das Verfahren hat besondere Bedeutung für die N,N-Dialkylierung von Anilin und dessen kernsubstituierten Derivaten. Als Substituenten des Anilins seien Halogene wie Fluor, Chlor und Brom, C₁-C₆-Alkyl- und Alkoxygruppen sowie die Nitro- und Cyanogruppe genannt. Auch mehrere dieser Substituenten sind möglich.

Besonders bevorzugte Ausgangsstoffe sind Anilin, o-, m-, p-Toluidin, die Xylidine und Anisidine sowie halogensubstituierte Aniline.

Als Alkohole (IIIa) können vorzugsweise geradkettige oder verzweigte C₁-C₁₀-Alkanole Verwendung finden,; insbesondere eignen sich Methanol und Ethanol. Daneben kommen Alkenole, Cycloalkanole und araliphatische Alkohole wie Benzylalkohol in Betracht.

Als Dialkylether (IIIb) können Ether mit Alkylresten mit 1 bis 6 C-Atomen eingesezt werden, wobei Ether mit gleichen Alkylresten besonders bevorzugt sind. Von besonderer Bedeutung sind Dimethyl- und Diethylether.

Im allgemeinen setzt man den Alkohol (IIIa) bzw. den Dialkylether (IIIb) im Überschuß über die stöchiometrisch erforderliche Menge ein, wobei bevorzugt je Mol Arylamin (II) vorteilhafterweise 1 bis 10 mol, insbesondere 1 bis 4 mol Alkylierungsmittel verwendet werden. Der nicht umgesetzte, überschüssige Alkohol oder Dialkylether läßt sich zurückgewinnen und wiederverwenden, nachdem das gegebenenfalls darin enthaltene, durch die Umsetzung entstandene Wasser abgetrennt ist.

Die Menge des aus der Sauerstoffsäure des Phosphors und Arylamin (IV) aufgebauten Katalysators ist nicht kritisch, beträgt aber vorzugsweise mindestens 0,01 kg pro kg stündlich zur Reaktion gebrachtem Arylamin (II). Vorzugsweise arbeitet man mit 0,05 bis 0,1 kg/kg Arylamin und Stunde des Katalysators, wogegen größere Mengen in der Regel keine Vorteile mehr bieten.

Die Alkylierung des Arylamins (II) findet bei einer Temperatur zwischen 180 und 250°C, vorzugsweise zwischen 200 und 230°C statt. Unterhalb von 180°C verlangsamt sich die Umsetzung zu sehr bzw. je nach Wahl der Ausgangsstoffe liegen diese nicht mehr gasförmig vor. Oberhalb von 250°C ist mit Kernalkylierungsreaktionen zu rechnen.

Die Umsetzung findet bei schwach erhöhtem Druck bis zu etwa 2 bar statt, gegebenenfalls kann die Reaktion auch bei Normaldruck durchgeführt werden.

Die erfindungsgemäße Katalysatorherstellung weist gegenüber dem Stand der Technik den Vorteil auf, daß die Bildung von Nebenprodukten, insbesondere von N-Monoalkylarylaminen, durch die Umsetzung der Sauerstoffsäure des Phosphors mit einem Arylamin (IV) vor Beginn der Alkylierungsreaktion verringert wird. Ein zusätzlicher Rückgang des Anteils an Nebenprodukten erfolgt bei der Umsetzung mit dem durch das Verfahren herstellbare N,N-Dialkylarylamin (I), was besonders vorteilhaft ist, so daß eine destillative Aufarbeitung wesentlich erleichtert wird oder entfallen kann.

Bei der Herstellung von N,N-Dimethylanilin wird durch die Vorbehandlung der Sauerstoffsäure des Phosphors mit Anilin oder N,N-Dimethylanilin die Selektivität gesteigert. Ferner stellt sich durch die Behandlung mit N,N-Dimethylanilin bereits sehr früh eine konstante Zusammensetzung in der Rohproduktphase ein.

Durch Vorbehandlung der Sauerstoffsäure des Phosphors mit einem Arylamin (IV) hat es sich als äußerst vorteilhaft erwiesen, die Reaktion von Zeit zu Zeit zu unterbrechen, um die Katalysatorphase mit Wasserdampf oder Wasser bei 100 bis 300°C zu behandeln. Dabei ist es vorteilhaft, die Behandlung bei einer Temperatur über 150 bis 300°C, insbesondere bei 180 bis 250°C durchzuführen.

Die Dauer des Durchleitens des Wasserdampfes oder das Einleiten des Wassers erfordert in der Regel 0,1 Stunden, vorzugsweise 0,3 bis 3 Stunden. Besonders bevorzugt sind 0,5 bis 2 Stunden. Ferner hat es sich als zweckmäßig erwiesen, durch die Katalysatorphase eine Dampfmenge von 0,1 bis 400 kg pro 1 m³ Katalysator und Stunde zu leiten. Besonders vorteilhaft ist eine Dampfmenge von 1 bis 300 kg, insbesondere von 10 bis 200 kg pro 1 m³ Katalysator und Stunde. An Stelle von Wasserdampf kann auch Wasser eingeleitet werden.

Nach der Behandlung mit Wasserdampf kann durch die Katalysatorphase Stickstoff geleitet werden, um überschüssiges, nicht mehr durch den Katalysator aufgenommenes Wasser auszutreiben. Diese Maßnahme ist nicht unbedingt erforderlich, da das Wasser auch durch die Wiederaufnahme der Synthese mit dem Produktstrom aus dem Reaktor ausgetragen werden kann.

Die Regenerierung des Katalysators des erfindungsgemäßen Verfahren weist gegenüber dem Stand der Technik den Vorteil auf, daß durch die Behandlung mit Wasserdampf oder Wasser die Produktivität des Katalysators erhalten bleibt und der Durchsatz an Ausgangsstoffen nicht reduziert werden braucht. Durch den von Zeit zu Zeit eingeleiteten Wasserdampf werden vermutlich leichtflüchtige phosphororganische Verbindungen, die durch Reaktionen des Katalysators mit den Edukten entstanden sind, hydrolysiert, und die Sauerstoffsäure des Phosphors wird wieder zurückgebildet.

Vorversuche hatten ergeben, daß nach einer Betriebsdauer von mehr als 2 Monaten leichtflüchtige phosphororganische Substanzen mit dem gasförmigen Reaktoraustrag mitgeschleppt werden, die dann in den nachgeschalteten Apparaturen starke Korrosionen und daran gekoppelt erhöhte Produktionskosten verursachen.

N,N-Dialkylarylamine (I), die nach den erfindungsgemäßen Verfahren hergestellt werden, sind wertvolle Zwischenprodukte für organische Synthesen, u.a. zur Herstellung von Pflanzenschutzmitteln, Arzneimitteln und Farbstoffen.

Besondere Bedeutung haben das erfindungsgemäße Katalysatorherstellungs- und Regenerierungsverfahren für die Herstellung von N,N-Dimethylanilin aus Anilin und Methanol sowie für die Herstellung von N,N-Diethylanilin aus Anilin und Ethanol.

### Beispiele 1 bis 5

### Herstellung von N,N-Dimethylanilin

Ein zylindrischer Reaktor von 3 m³ Inhalt, der mit 1 m³ Orthophosphorsäure beschickt worden war, wurde auf eine Temperatur von 210°C erhitzt. Dadurch freigesetztes Kondensationswasser der Orthophosphorsäure wurde zunächst mittels eines Stickstoffstroms aus dem Reaktor ausgetragen. Anschließend wurde gasförmiges Anilin bzw. gasförmiges N,N-Dimethylanilin in die so hergestellte Sauerstoffsäure des Phosphors geleitet, bis jeweils das aus der Tabelle zu entnehmende molare Verhältnis von Stickstoff zu Phosphor erreicht war. Dann wurde stündlich bei einer Temperatur von 228°C ein gasförmiges Gemisch aus 130 kg Anilin und 177 kg Methanol (Molverhältnis 1:4) durch den Reaktor geleitet, wobei die Verweilzeit der Edukte jeweils 50 Sekunden betrug.

Der Reaktoraustrag wurde kondensiert, und nicht umgesetztes Methanol wurde destillativ entfernt. Nach Abtrennung der im Sumpf vorhandenen wäßrigen Phase wurde die Rohproduktphase gaschromatographisch analysiert.

### Beispiel 6

### Herstellung von N,N-Diethylanilin

Die Durchführung erfolgte entsprechend den Beispielen 1 bis 5. Anstelle des Gemisches aus Anilin und Methanol wurde ein gasförmiges Gemisch aus 100 kg Anilin und 195 kg Ethanol (Molverhältnis 1:4) durch den Reaktor geleitet.

### Beispiel 7 (Vergleichsbeispiel)

Die Durchführung erfolgte entsprechend den Beispielen 1 bis 5, jedoch entfiel die Umsetzung der Sauerstoffsäure des Phosphors mit dem Arylamin.

Die Ergebnisse der Beispiele 1 bis 7 sind der Tabelle zu entnehmen.

Sie zeigen, daß gegenüber dem Stand der Technik durch die Behandlung der Sauerstoffsäure des Phosphors mit Arylamin (IV), insbesondere mit N,N-Dimethylanilin die Selektivität bei der Herstellung von N,N-Dimethylanilin nach dem erfindungsgemäßen Verfahren gesteigert und der Anteil an Nebenprodukten im Rohprodukt, insbesondere der Anteil an N-Monomethylanilin, verringert wird.

### Beispiel 8

Ein zylindrischer Reaktor von 3 m³ Inhalt, der mit 1 m³ Orthophosphorsäure beschickt worden war, wurde auf eine Temperatur von 230°C erhitzt. Dadurch freigesetztes Kondensationswasser der Orthophosphorsäure wurde zunächst mittels eines Stickstoffstroms aus dem Reaktor ausgetragen. Anschließend wurde solange gasförmiges Anilin durch die so hergestellte Sauerstoffsäure des Phosphors geleitet, bis sie kein Anilin mehr aufnahm, und es im Reaktoraustrag nachweisbar war. Dann wurde stündlich ein gasförmiges Gemisch aus 130 kg Anilin und 177 kg Methanol (Molverhältnis 1:4) durch den Reaktor geleitet. Der Reaktoraustrag wurde kondensiert und nicht umgesetztes Methanol destillativ entfernt. Nach Abtrennung der im Sumpf vorhandenen wäßrigen Phase wurde die Rohproduktphase einer Fraktionierkolonne zugeleitet.

Die gaschromatographischen Analysen des Rohproduktes und des destillierten Produktes lieferten folgende Ergebnisse:

### Zusammensetzung des Rohproduktes:

98,55 Gew.-% N,N-Dimethylanilin,
0,95 Gew.-% N-Monomethylanilin,
0,5 Gew.-% o,p-Toluidine und
0,001 Gew.-% Anilin
Das destillierte Produkt enthielt 99,8 Gew.-% N,N-Dimethylanilin.

Im Laufe von 60 Tagen sank die Produktivität infolge der Desaktivierung der Katalysatorphase um 50 %, so daß der Durchsatz von Anilin und Methanol auf 65 kg/h bzw. 88 kg/h reduziert werden mußte. Die Reaktion wurde unterbrochen und die Zufuhr der Ausgangsstoffe abgeschaltet, um danach bei 230°C zwei Stunden lang 50 kg Wasserdampf mit einem Druck von 4 bar durch den Katalysator zu leiten.

Durch diese Maßnahme wurde die ursprüngliche Aktivität der Katalysatorphase wieder hergestellt, so daß die stündliche Einleitung des gasförmigen Gemisches bestehend aus 130 kg Anilin und 177 kg Methanol fortgesetzt werden konnte.

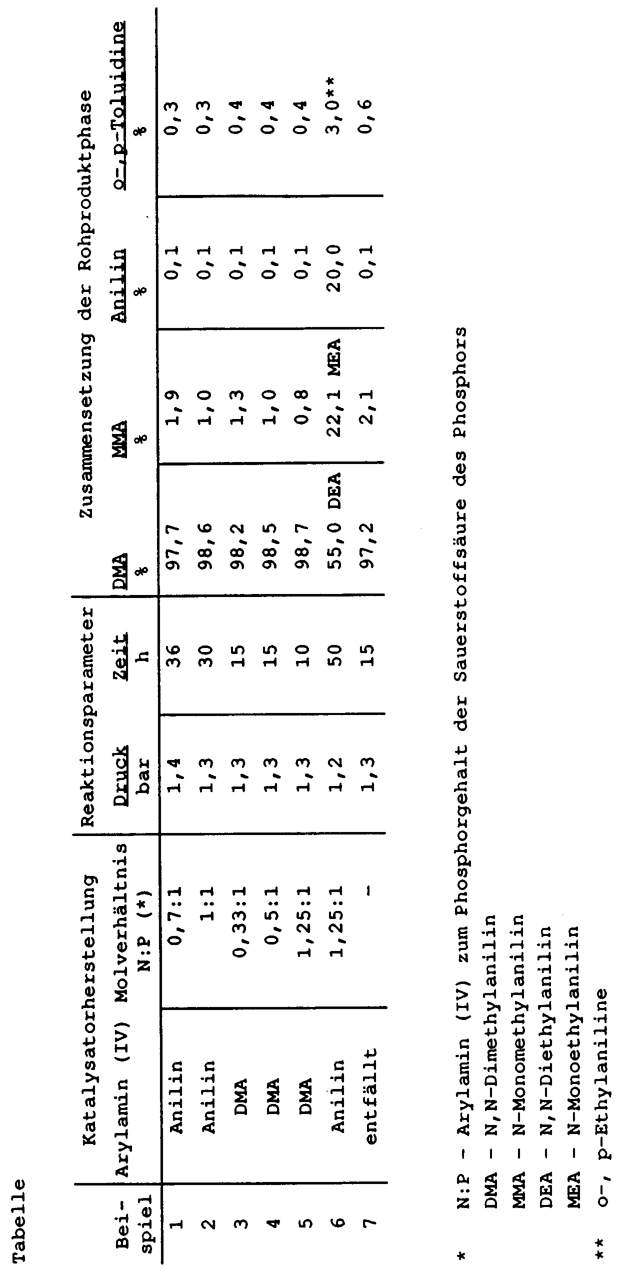

## Patentansprüche

1. Verfahren zur Herstellung von N,N-Dialkylarylaminen (I) durch Umsetzung von Arylaminen (II) mit Alkoholen (IIIa) oder Dialkylethern (IIIb) in Gegenwart einer Sauerstoffsäure des Phosphors oder eines Ammoniumsalzes hiervon als Katalysator, dadurch gekennzeichnet, daß man die Sauerstoffsäure des Phosphors oder das Ammoniumsalz hiervon vor Beginn der Reaktion mit einem Arylamin (IV) umsetzt.

2. Verfahren nach Anspruch 1, dadurch gekennzeichnet, daß man hierbei als Arylamin (IV) eines der Amine (I) oder (II) verwendet.

3. Verfahren nach Anspruch 1 oder 2, dadurch gekennzeichnet, daß das Molverhältnis von Arylamin (IV) zum Phosphorgehalt der Sauerstoffsäure des Phosphors 0,1:1 bis 2:1 beträgt.

4. Verfahren nach Anspruch 1, dadurch gekennzeichnet, daß man die Reaktion von Zeit zu Zeit unterbricht und die Katalysatorphase bei 100 bis 300°C mit Wasserdampf oder Wasser behandelt.

5. Verfahren nach den Ansprüchen 1 bis 4, dadurch gekennzeichnet, daß man sie auf die Herstellung von N,N-Dimethylanilin aus Anilin und Methanol anwendet.

6. Verfahren nach den Ansprüchen 1 bis 4, dadurch gekennzeichnet, daß man sie auf die Herstellung von N,N-Diethylanilin aus Anilin und Ethanol anwendet.

## Claims

1. A process for the preparation of N,N-dialkylarylamines (I) by reacting arylamines (II) with alcohols (IIIa) or dialkyl ethers (IIIb) in the presence of an oxygen acid of phosphorus or in the presence of an ammonium salt thereof as catalyst, which comprises reacting the oxygen acid of phosphorus or the ammonium salt thereof with an arylamine (IV) before the reaction is commenced.

2. A process as claimed in claim 1 or 2, wherein the arylamine (IV) used is one of the amines (I) and (II).

3. A process as claimed in claim 1 or 2, wherein the molar ratio between the arylamine (IV) and the phosphorus content of the oxygen acid of phosphorus is from 0.1:1 to 2:1.

4. A process as claimed in claim 1, which comprises occasionally interrupting the reaction and treating the catalyst phase at from 100 to 300°C with steam or water.

5. A process as claimed in claims 1 to 4, wherein N,N-dimethylaniline is prepared from aniline and methanol.

6. A process as claimed in claims 1 to 4, wherein N,N-diethylaniline is prepared from aniline and ethanol.

## Revendications

1. Procédé de préparation de N,N-dialkylarylamines (I) par la réaction d'arylamines (II) avec des alcools (IIIa) ou des éthers dialkyliques (IIIb) en présence d'un acide oxygéné du phosphore ou d'un sel d'ammonium de celui-ci à titre de catalyseur, caractérisé en ce que l'on fait réagir l'acide oxygéné du phosphore ou le sel d'ammonium de celui-ci avec une arylamine (IV) avant le début de la réaction.

2. Procédé suivant la revendication 1, caractérisé en ce que l'on utilise à cette fin, à titre d'arylamine (IV), l'une des amines (I) et (II).

3. Procédé suivant la revendication 1 ou 2, caractérisé en ce que le rapport molaire de l'arylamine (IV) à la teneur en phosphore de l'acide oxygéné du phosphore varie de 0,1:1 à 2:1.

4. Procédé suivant la revendication 1, caractérisé en ce que l'on interrompt la réaction de temps en temps et on traite la phase du catalyseur à 100-300°C par de la vapeur d'eau ou de l'eau.

5. Procédé suivant l'une quelconque des revendications 1 à 4, caractérisé en ce qu'on le met en oeuvre en vue de la préparation de la N,N-diméthylaniline à partir de l'aniline et du méthanol.

6. Procédé suivant l'une quelconque des revendications 1 à 4, caractérisé en ce qu'on le met en oeuvre en vue de la préparation de la N,N-diéthylaniline à partir de l'aniline et de l'éthanol.
